# EUROPEAN PATENT APPLICATION

(11) **EP 2 508 894 A1**
(43) Date of publication of application: **10.10.2012**
(21) Application number: 11003712.4
(22) Date of filing: 05.05.2011
(51) Int. Cl.: G01N 33/92

(54) **Method for monitoring gene expression of translation and Integral/Secretory protein synthesis by magnetic resonance spectroscopy (MRS)**

(30) Priority: 05.04.2011 US 471971 P
(71) Applicant: Receptomon LLC, Glenview IL 60026 (US)
(72) Inventor: Norfray, Joseph, F., Glenview, Illinois 60026 (US)
(74) Representative: Pfenning, Meinig & Partner GbR

(57) **Abstract**

Disclosed is a method for monitoring change in gene expression of translation and integral/secretory protein synthesis in a sample comprising measuring the amount of Choline and/or a fatty acid present in the sample as a function of time wherein the measurement is carried out by Magnetic Resonance Spectroscopy (MRS) and correlating the amount of Choline and/or fatty acid measured as function of time to the change in gene expression of translation and integral/secretory protein synthesis in the sample. The level of Choline from organelles matches the level of Choline from transcription and integral/secretory protein synthesis. Quantification of translation and integral/secretory protein synthesis leads to quantification of genetic expression. During apoptosis the amount of Choline decreases, and the amount of fatty acids increase from distinct trafficking of Choline polar heads and fatty and tails of degraded endomembranes.

## Description

### BACKGROUND OF THE INVENTION

Gene expression represents the active genes controlling cell growth, proliferation and survival. Three macromolecules contain a person's genetic code - deoxyribonucleic acid (DNA), ribonucleic acid (RNA), and proteins Chapter 17, "Gene expression: the genetic code and transcription", in The World of the Cell. 3rd ed, Becker, WM. et al., (Eds), Benjamin/Cummings Publishing Company (1996). With few exceptions, each cell in the human body contains the same complement of genes. The repression and activation of different combinations of genes determines growth, proliferation and survival of a cell. The activity of a gene is regulated by a receptor located either on the cell surface, or within the cell. A specific molecule activates specific receptors, initiating specific transcription factor, to activate a specific gene on DNA. The specific gene on the DNA becomes a template forming a specific messenger RNA (mRNA) through transcription. The mRNA leaves the nucleus generating a specific protein in the cytoplasm through translation. The protein, in turn, becomes a component of a signaling pathway regulating growth, proliferation and survival.

Methods for monitoring gene expression utilize quantification of the three macromolecules (DNA, RNA, and protein) containing the genetic code. Various methods have been developed including: active DNA identified by a monoclonal antibody; active mRNA identified by DNA arrays; and the presence of a protein identified by a monoclonal antibody.

In 1983, a method for monitoring active DNA associated with cell proliferation was described Gerdes, J. et al., Int J Cancer 31:13-20 (1983). Recently, MRS has correlated increase cell proliferation (Ki-67 labeling index) with increase level of choline, a metabolite specific to cell membranes. Shimizer, H. et al., AJNR 21:659-665 (2000); Herminghaus, S. et al., NMR Biomed 15:385-392 (2002); Shukia-Dove, A. et al., Radiology 250:803-809 (2009). MRS has been shown as a robust method to quantify the level of cell membranes, by quantifying the level of choline. Podo, F. NMR Biomed 12:413-439 (1999), Bluml, S. Magn Reson Med 42:643-654 (1999). Since cell proliferation implies increase in cell density (number), the increase in choline seen on MRS was incorrectly assumed to arise from the increase in plasma membranes. Because the MRS articles correlated with the cell proliferation the importance of internal cell membranes was overlooked. Shimizer, H. et al., *supra,* Herminghaus, S. et al., *supra,* Shukia-Dove, A. et al., *supra,* never suggested the increase in choline was from intracellular membranes.

DNA arrays quantify gene expression by measuring the amount of mRNA bound to each site on the DNA array. Many DNA genes are arranged in regulate immobile positions. Known and unknown samples of mRNA undergo reverse transcription forming complementary DNA (cDNA), then are fluorescently labeled, added to the array, with the mRNA (cDNA) binding to the DNA base pairs through hybridization. Lasers activate the fluorescent dyes bound to active genes with the level of fluorescent signal intensity indicating the level of gene activity Young, RA. Et al., Cell 102:9-15 (2000); Eisen, MB. et al., Proc Natl Acad Sci 96:10943-10944 (1999). The DNA arrays utilize nucleic acids that become hybridized, therefore, quantifying the intra nuclear macromolecules of gene expression. The DNA arrays do not quantify the cytoplasmic macromolecule, protein. Therefore, the molecular pathways of translation and protein synthesis are not investigated by DNA arrays. DNA arrays do not quantify intracellular membranes.

Gene expression can be quantified by measuring the level of a protein identified by monoclonal antibodies against a specific protein antigen. Two important protein antigens are prostate specific antigen (PSA), and carcinoembryonic antigen (CEA). Elevated PSA and CEA indicate elevated proteins seen with prostate cancer (PSA), and gastrointestinal cancers (CEA). Both tests require a blood sample. Quantification of a specific protein by immunohistochemistry does not quantify intracellular membranes.

MRS is a valuable tool identifying and quantifying the choline and phosphorus metabolites of cell membranes Daly, F. et al., J Biol Chem 262:14975-14973 (1987); Ruiz-Cabello, J. et al., NMR Biomed 5:226-233 (1992); Podo, F. et al., *supra;* Bluml, S. et al., *supra.* Identification and quantification the MRS metabolites of cell membranes allowed the investigation of the chemical pathways of cell membranes however, MRS could not overcome the hurdle of distinguishing between intracellular and plasma membranes. The ¹H MRS of choline represents the sum of both the intracellular and plasma membranes. Bluml, S. et al., *supra.* Because the source of the choline signal from intracellular and plasma membranes were not distinguished, quantification of intracellular membranes, utilizing chemical pathways was impossible.

The foregoing shows that there exists an unmet need for monitoring gene expression of translation and integral secretory protein synthesis.

### BRIEF SUMMARY OF THE INVENTION

The invention provides a method for monitoring change in gene expression of translation and integral/secretory protein synthesis in a sample comprising measuring the amount of Choline and/or a fatty acid present in the sample as a function of time wherein the measurement is carried out by Magnetic Resonance Spectroscopy (MRS) and correlating the amount of Choline and/or fatty acid measured as function of time to the change in gene expression of translation and integral/secretory protein synthesis in the sample.

### DETAILED DESCRIPTION OF THE INVENTION

The present invention is predicated on monitoring gene expression of translation and total protein synthesis by quantifying Choline, which is a bioassay of intracellular membranes with MRS. Choline is a common thread linking the chemical, biologic and genetic pathways of translation and intregral/secretory protein synthesis. Quantification of translation and intregral/secretory protein synthesis quantifies genetic expression.

MRS is a tool for identifying and quantifying membrane metabolites. A nucleus with unpaired protons and/or neutrons is magnetized when placed in an external magnetic field. The nucleus absorbs radiofrequency energy at its Larmor frequency. The emitted frequency of the nucleus is altered by metabolite magnetic fields from both the metabolite's electrons (chemical shift), as well as, neighboring nuclei (J coupling). Every membrane metabolite is composed of specific electrons and nuclei, thereby, generating a specific energy frequency. During MRS all free, and mobile membrane metabolites and their frequencies are visualized. The frequencies are separated into their distinct peak frequencies by Fourier transformation. The height of the peaks indicates the quantity of the membrane metabolites. Chapter 1, "Introduction to MR spectroscopy in vivo". in Clinical MR Spectroscopy Techniques and Applications, 1st ed, Barker, PB, et al., (Eds), Cambridge University Press (2010).

Specific nuclei are found within membranes, thereby, visualizing and quantifying metabolites of cell membranes by MRS. The ¹H, ¹³C, ¹⁵N and ³¹P are found in the Choline polar heads of phospholipids. The ¹H, and ¹³C are found in the fatty acid tails of phospholipids. ³¹P identified and quantified phosphomonoesters, the chemical metabolites of membrane synthesis, and phosphodiesters, the chemical metabolites of membrane degradation Ruiz-Cabello, J. et al., *supra.* ¹H has been able to assign the total visible Choline to a single peak at 3.20 ppm, including, the free, and mobile membrane bound Choline. Bluml, S. et al., *supra.* The ¹H MRS Choline peak is a bioassay of cell membranes.

All the visible MRS nuclei provide distinct and valuable information about membranes, with a recent emphasis on ¹H nucleus because of increase of ¹H high sensitivity from its high natural abundance and high gyro magnetic ratio. The Choline signal is amplified nine-fold in the ¹H MRS spectrum, with Choline containing 9 protons per molecule as trimethylamines, or trimethylamine salts. The Choline signal is amplified 10,000 fold by suppression of the water peak in the ¹H MRS spectrum.

All membranes are phospholipids containing cholesterol, integral proteins and glycolipids. Phospholipids are composed of Choline polar heads, and non-polar fatty acid tails. The polar heads are hydrophilic, while the fatty acid tails are hydrophobic. When phospholipids are exposed to aqueous solutions the phospholipids assemble into one of three structures - a lysosomes (a closed bilayer), a micelle (a closed unilayer), or membranes (an open bilayer, i.e., plasma and intracellular membranes). The polar heads interact with water molecules, while the non-polar fatty acid tails interact with adjacent fatty acid tails. The Choline polar heads and fatty acid tails are chemically joined by either a glycerol or sphinogosine backbone.

All phospholipids contain Choline polar heads. The only other metabolite of Choline is acetylcholine, a neurotransmitter, in sub-millimolar concentrations, and not visualized by MRS. Therefore, the total Choline peak in MRS arises from phospholipids membranes. The ¹H MRS Choline peak is a bioassay of phospholipids membranes. The term "Choline" herein is used to denote choline ((CH₃)₃N⁺CH₂CH₂OH), a derivative of choline, or a combination of choline and/or one or more derivatives of choline (e.g., phosphocholine), phosphodiesters of choline (e.g., phosphatidylcholine, sphingomyelin, phosphoethanolamine, glycerophosphoethanolamine, or any combination thereof. In an embodiment of the invention, the term "Choline" represents the sum of choline and all choline derivatives (or total choline), for example, the sum of choline and phosphocholine. Phosphoserine and glycerophosphoserine also can be monitored Ruiz-Cabello, J. et al., NMR in Biomedicine, 5, 223-233 (1992); Podo, F., NMR in Biomedicine, 12, 413-439 (1999); and Blums, S. et al., Magn Reson Med, 42, 643-654 (1999).

All phospholipids contain fatty acid tails. Fatty acid tails are composed of two fatty acids with one unsaturated, and the other saturated. Fatty acids are components of membranes. Fatty acids are also components of lipid droplets where fatty acids are stored as a future source of energy. In contra-distinction to Choline, which is limited to the polar head of membranes allowing quantification of membranes, fatty acids are components of both membranes and lipid droplets, thereby preventing quantification of membranes.

In accordance with an embodiment of the invention, the term saturated fatty acids is used to denote the ¹**H** chemical shift of the C**H**₂ group at 1.3 ppm, for example, as in - (C**H**₂)ₙ-, where n represents the number of carbon atoms in the fatty acid, e.g., n is commonly 16 or 18, *Molecular Cell Biology,* supra. The term "unsaturated fatty acids" is used to denote, for example, the ¹**H** chemical shift of the C**H**₂ group at about 2.8 ppm, for example, as in -(CH=CH-CH₂-CH=CH)-, and/or the ¹**H** chemical shift of the unsaturated methinyl group -(C**H**=C**H**)- occurring at about 5.4 ppm. Alternatively, ¹³C resonances can be monitored for these groups. The ¹³**C** chemical shifts of the CH₂ group of saturated fatty acids are about 20.5, about 22.8, about 24.7, about 29.2-29.7, about 31.4, about 32 and about 34 ppm. ¹³C chemical shift of the inner double bonds - (C**H**=C**H**-**C**H₂-**C**H=C**H**) - group of unsaturated fatty acids is about 128 ppm. ¹³**C** chemical shift of the outer double bonds - (**CH**=**CH**) - and -(CH=CH-CH₂-CH=CH)- group of unsaturated fatty acids is about 130 ppm.

A modem day Rosetta stone is monitoring gene expression of translation and integral/secretory protein synthesis by quantifying the Choline of intracellular membranes with MRS. The basis of the patent is Choline, a common thread, linking the chemical, biologic and genetic pathways of translation and integral/secretory protein synthesis. Advancements in molecular biology identified 90% of all cell membranes are intracellular, the organelles Encyclopaedia Britannica, "Cell: Their structure and functions", The New Encyclopaedia Britannica, V. 15, Macropaedia, 15th edition, pp 565-593 (1994). No previous "person of ordinary skill" in MRS suggested intracellular membranes as the major source of membranes. Since 90% of membranes are intracellular, 90% of the Choline peak is linked to organelles.

Advancements in biologic pathways identified the endoplasmic reticulum (ER) as the site of translation and integral/secretory protein synthesis. Translation for integral and secretory proteins occurs on the surface of the ER by membrane bound ribosomes. The membrane bound ribosomes are the workbenches of translation utilizing messenger RNA (mRNA) as a template to generate the correct amino acid (AA) sequence in the polypeptide. The nascent AA sequence leaves the ribosome and enters the lumen of the ER. Protein synthesis occurs within the lumen of the ER, and includes: elongation of the polypeptide; addition of sulfur and glycolipid side chains; and folding. The other organelles have functions supporting translation and protein synthesis. Golgi apparatus is the site for sorting integral and secretory protein. Lysosomes are the site for degrading unfolded proteins. Peroxisomes are the site for oxidizing hydrogen peroxide generated during protein synthesis. Transport and secretory vesicles are the vehicles of transporting and secreting proteins. Finally, the pores of the nuclear intracellular membrane transport the components of RNA for translation in the cytoplasm. Since the biologic pathway of translation and integral/secretory protein synthesis are on or within the intracellular membranes, Choline is linked to translation and integral/secretory protein synthesis. Chapter 5, "Biomembranes and cell architecture", in Molecular Cell Biology, 5th ed., Lodish, H., et al. (Eds), W. H. Freeman and Co (2004).

Advancements in genetic pathways identified genes regulate the level of organelles to match the level of translation and integral/secretory protein synthesis. The unfolded protein response (UPR) is an intracellular signaling pathway between the endoplasmic reticulum (ER) and DNA. Three receptors in the lumen of the ER sense accumulation of unfolded proteins thereby, releasing transcription factors expanding the abundance of organelles to maintain the fidelity of protein folding Bernales, S. et al., Ann Rev Cell Biol 22:487-508 (2006). Proliferation of the ER is rapid, doubling in 40 minutes Bernales, S. et al., *supra.* Autophagia genes are also induced by the UPR, and are essential for survival of cells subject to sever ER stress Bernales, S. et al., Plos Biol 4 (e 423) 2311-2347 (2006). Under severe cellular stress, translation initiation factors are blocked in the early commitment phase of apoptosis preventing the assembly of ribosomes, and thereby blocking translation Clemens, MJ. et al., Cell Death Differ 7:603-615 (2000). Apoptosis, programmed cell death reduces protein synthesis 60-70% within several hours Clemens, MJ. et al., *supra.* Since genetic pathways regulate the level of intracellular membranes to match the level of translation and integral/secretory protein synthesis, Choline is linked to the level of translation and integral/secretory protein synthesis. Therefore, the method monitors early gene expression of translation and integral/secretory protein synthesis by monitoring the choline of intracellular membranes with MRS.

During apoptosis, the intracellular membranes are degraded, with release of Choline polar heads, and fatty acid tails. The rapid rise of the fatty acid MRS peaks, combined with a decrease in the Choline MRS peak is a biologic marker of apoptosis. The trafficking of the Choline polar heads and the saturated/unsaturated fatty acid tails of degraded endomembranes explains why Choline decreases and saturated/unsaturated fatty acid tails increases during apoptosis. Choline, a small molecular essential nutrient, normally enters the cell by diffusion and by plasma membrane organic cation transporters. During apoptosis, Choline decreases from both reduced active Choline transport, and diffusion of Choline out through the plasma membrane. Fatty acid tails are large chains, and are retained by intact plasma membrane as lipid droplets and within lysosomes; Kauppinen et al, *supra.*

A possible reason why the saturated/unsaturated fatty acid peaks increase before the Choline peak decreases is the continued active transport of Choline into the cell until energy deprivation is complete. The invention identifies the decrease Choline, and the increase in fatty acids during apoptosis by monitoring intracellular membranes.

The amount of Choline can be measured by MRS in any suitable manner. For example, in an embodiment, the amount of Choline can be measured by measuring the height of a peak corresponding to Choline. In another embodiment, the amount of Choline can be measured by measuring the area under a peak corresponding Choline. In yet another embodiment, the amount of Choline can be measured by measuring the ratio of the height of a peak corresponding to Choline relative to the height of a peak of an internal or external standard. In a further embodiment, the amount of Choline can be measured by measuring the ratio of the area under a peak corresponding to Choline relative to the area under a peak of an internal or external standard.

Any suitable internal or external standard can be used. For example, the internal standard is total creatine when the MRS is based on ¹H resonance. The term "total creatine" refers to the combination of creatine and phosphocreatine. Creatine is buffered in cell systems; accordingly, the amount of creatine remains substantially constant. Examples of external standards are Electronic reference to access in vivo concentrations (ERETIC) and 3-(trimethylsilyl) propionic -2, 2, 3-d₄ acid (TSP). Alber MJ, et al., Magn Reson Med 61, 525-532 (2009).

It is contemplated that the present inventive method is applicable to monitoring early gene expression during apoptosis. In accordance with an embodiment of the present invention apoptosis causes an interruption in an up-regulated intracellular organelle; for example, an interruption in the function of the secretory granules and/or the transporting vesicles. In accordance with another embodiment of the invention apoptosis causes an interruption in the function of the Golgi apparatus. In further embodiments of the invention causes an interruption in the function of the lysosomes, the endoplasmic reticulum, the mitochondrion, the nucleus, and/or the peroxisomes.

In accordance with the present inventive method, gene expression can be measured initially, e.g., at any given time, and then within a period of about 168 hours, preferably about 24 hours, and more preferably about 12 hours. For example, the gene expression can be monitored every 12, 24, 36, 48, 60, 72, 84, 96, 108, 120, 132, 144, 156, or 168 hours, or any combination thereof, after the initial measurement.

Gene expression can also be documented using the current invention within 24 to 168 hours by monitoring translation. This embodiment utilizes a magnetic resonance magnet, equal to or greater than 0.5 Tesla, uses existing software and coils which are commercially available, has high spatial resolution, lacks radiation, employs user-friendly automatic sequences, allows non-invasive sequential analysis and provides quantification from multiple MR sites. This embodiment can monitor gene expression in vivo and in vitro, in humans and laboratory animals, as well as, in tissues and perfused cell extracts. This embodiment quantifies normal translation, as well as the amplified translation. Since protein translation occurs in all cells, all histologies can be studied.

The present invention also provides a method for monitoring gene expression comprising: (a) localizing a tissue in a patient; (b) selecting a region of interest (ROI) of the tissue; (c) obtaining magnetic resonance spectra (MRS) of the ROI; (d) measuring the amount of Choline from the MRS spectra; (e) obtaining MR spectra of the tissue at the same ROI within a period of 7 days, preferably 3 days, and more preferably within 1 day, (f) measuring the amount of Choline from the MR spectra; and (g) comparing the amount of Choline obtained initially with the amount of Choline obtained later; whereby increase in the amount Choline is indicative of increase gene expression; decrease in the amount of Choline is indicative of decrease in gene expression; and lack of change in the amount of Choline is indicative of a lack of change in gene expression.

The basis for clinical MR studies (e.g., MRS or MRI) is a nucleus, with a positive charge, for example, the hydrogen nucleus--the proton. The same machinery is used for MRI and MRS. They differ in the software manipulation of the emitted radiofrequency (RF) from the ¹H nuclei. In MRI, the signal is used to create the image; in MRS, the signal is used to create the spectrum. Fourier Transform principle allows the MRS software to separate the individual RFs within the signal. The spectrum therefore represents the different RFs being emitted within the selected region of interest (ROI). The points along the horizontal axis of the spectrum represent specific RFs emitted from each metabolite. The vertical axis of the spectrum is proportional to the amount of each metabolite forming the area beneath the RF peaks. Spectra can be obtained on 0.5 T and higher MR scanners, although high-field strength scanners provide better definition of the spectra. Spectra obtained with different-strength scanners can be compared on a scale in parts per million (ppm) along the horizontal axis because metabolites always reside at one or more specific sites, for example, in 1H MRS, Choline reside at 3.20 ppm.

Any suitable MR spectrometer can be used in the practice of the present invention. Clinical MR spectra can be obtained on MR scanners, for example, utilizing the clinical spectroscopy package called proton brain exam/single voxel (PROBE/SV) developed by General Electric Medical Systems (Milwaukee, WI) for use with GE's 1.5 Tesla (T) MR scanner. See Norfray, J. et al., AJR 173, 119-125 (1999), Norfray, J. et al., J Comput Assist Tomogr 23, 994-1003 (1999), and Norfray, J.F. et al., "Magnetic resonance spectroscopy" in Pediatric Neurosurgery, 4th ed. McLane, DG, (Ed), WB Saunders (2001) for procedures for obtaining MR spectra, identification of the peaks corresponding to metabolites such as Choline, creatine, and others, and ratio of the peaks. See also Danielsen and Ross, Magnetic Resonance Spectroscopy Diagnosis of Neurological Diseases, Marcel Dekker, Inc. (1999); Ross, B. et al., Magnetic Resonance Quarterly, 10, 191-247 (1994); and Ross et al., U.S. Patent 5,617,861. Based on the information in the above publications, as well as information available in the art, those of skill in the art should be able to practice the invention on all types of tumors in accordance with the present invention.

The present invention can be carried out in any suitable manner, for example, as follows. Prior to MRS on a patient, the tissue is localized. Thus, for example, magnetic resonance images (MRI) of the tissue, e.g., brain, breast, or bone, with axial, sagittal, and coronal T1 and T2 images are obtained with and without contrast. A region of interest (ROI) of tissue is selected. This can be carried out based on the MRI findings to determine the tissue volume and location to be studied. MR spectra of the ROI are obtained within the tissue utilizing short and/or long TE (echo time) pulse sequences. The spectra obtained are interpreted. The peak corresponding to Choline is identified, e.g., at a chemical shift of 3.20 ppm. Based on the Choline peak, the amount of total cellular membranes is determined from either the height of the peak or the area under the peak. An internal or external standard is identified in the ROI. An example of an internal standard is creatine or total creatine. An example of an external standard is 100% 2-(trimethylsilyl) ethanol (TSE), which may be taped to the head coil of the MR spectrometer. Kreis, R. et al., J. Magnetic Resonance, Series B 102, 9-19 (1993). The ratio of the Choline to the standard is calculated. The Choline to creatine ratio represents a measure of the total cell membranes within the ROI of the tissue.

The gene expression can be monitored, for example, at 24 hours (day 1) to 168 hours (day 7), from any given time as follows. The tissue is localized utilizing the same MRI pulse sequences as initially. The same ROI is selected within the tissue. MR spectra of the tumor are obtained utilizing the same pulse sequences, the same TR (relaxation time), TE (echo time), phases, and frequency averages. The MR spectra are interpreted as before and the Choline to creatine ratio (e.g., height or area ratios) is calculated.

If the observed increase in the Choline to creatine ratio is 15% or more, preferably 20% or more, and more preferably 25% or more, then it can be concluded an increase in early gene expression has occurred. The early increase in the Choline to creatine ratio identifies a increase in the intracellular cell membranes, for example, a increase in the organelles and their granules and/or vesicles.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by contradicted by context.

## Claims

1. A method for monitoring change in gene expression of translation and integral/secretory protein synthesis in a sample comprising measuring the amount of Choline and/or a fatty acid present in the sample as a function of time wherein the measurement is carried out by Magnetic Resonance Spectroscopy (MRS) and correlating the amount of Choline and/or fatty acid measured as function of time to the change in gene expression of translation and integral/secretory protein synthesis in the sample.

2. The method of claim 1, wherein the MRS is based on the resonance of nuclei selected from the group consisting of ¹H, ¹³C, ¹⁵N, ³¹P, and any combination thereof, preferably wherein the nuclei is ¹H.

3. The method of any one of claims 1-2, comprising correlating the amount of Choline with the amount of intracellular membranes.

4. The method of claim 3, wherein the Choline is measured by measuring the height of a peak at δ 3.20 ppm by ¹H MRS.

5. The method of claim 1, wherein the fatty acid is a saturated fatty acid or an unsaturated fatty acid.

6. The method of claim 5, wherein the amount of saturated fatty is measured by ¹H MRS by measuring the height of a CH₂ peak at δ 1.3 ppm and/or the amount of unsaturated fatty is measured by measuring the height of a CH₂ group adjacent to a CH=CH group at δ 2.8 ppm or a CH=CH peak at δ 5.4 ppm or wherein the amount of fatty acid is measured by ¹³C MRS by measuring the height of the CH₂ peak at δ about 20.5, about 22.8, about 24.7, about 29-2-29.7, about 31.4, about 32, and/or about 34 ppm and/or of the inner double bonds peak of -(CH=**C**H-CH₂-**C**H=CH)- at δ about 128 ppm, or of the outer double bonds peak of-(**C**H=**C**H) - and -(CH=CH-CH₂-CH=CH)- at about 130 ppm.

7. The method of any one of claims 1-6, which correlates the amount of Choline to unfolded protein response, autophagy or apoptosis taking place in the sample.

8. The method of claim 4, which involves measuring the amount of free Choline or the amount of mobile Choline from endo membranes.

9. The method of any one of claims 1-8, wherein the amount of Choline or fatty acid is measured within a period of about 12 hours to within about 168 hours.

10. The method of any one of claims 1-9, wherein the sample is
● a tissue, preferably selected from the group consisting of brain, colorectal, breast, hematogenous, lung, kidney, squamous cell, testicular, stomach, mesenchymal, ovarian, esophageal, pancreatic, prostate, bone, and liver, or
● a cell, or
● a cell extract.

11. The method of any one of claims 1-10, wherein gene expression changes in an intracellular organelle or intracellular organelles selected from the group consisting of secretory granules, transporting vesicles, Golgi apparatus, lysosomes, endoplasmic reticulum, mitochondrion, nucleus, and/or peroxisomes.

12. The method of any one of claims 1-11, wherein, during apoptosis, the amount of Choline decreases, and the amount of fatty acids increase from distinct trafficking of Choline polar heads and fatty acid tails of degraded endomembranes.

13. A method for monitoring gene expression comprising: (a) localizing a tissue in a patient; (b) selecting a region of interest (ROI) of the tissue; (c) obtaining magnetic resonance spectra (MRS) of the ROI; (d) measuring the amount of Choline from the MRS spectra; (e) obtaining MR spectra of the tissue at the same ROI within a period of 7 days; (f) measuring the amount of Choline from the MR spectra; and (g) comparing the amount of Choline obtained initially with the amount Choline obtained later; and correlating increase in the amount of Choline with increase in gene expression; decrease in the amount of Choline with decrease in gene expression; and lack of change in the amount of Choline with lack of change in gene expression.

14. A method for monitoring translation and integral/secretory protein synthesis in a tissue comprising and measuring, by Magnetic Resonance Spectroscopy, the amount of Choline present in the intracellular membranes of the tissue initially and after an interval, obtaining a difference between the amounts Choline, and correlating the change to changes of protein translation and integral/secretory protein synthesis.

15. A method for quantifying the genetic pathways of apoptosis, autophagy and unfolded protein response by measuring non-invasively, by Magnetic Resonance Spectroscopy, the amount of Choline present in the tissue before and within a period of about 168 hours, obtaining a difference between the Choline amounts, and correlating the changes in the Choline amounts with changes in genetic pathways.
